# EUROPEAN PATENT SPECIFICATION

(11) **EP 0 211 647 B1**
(45) Date of publication and mention of the grant of the patent: **22.09.1993**
(21) Application number: 86306014.1
(22) Date of filing: 05.08.1986
(51) Int. Cl.: A61K 9/50, A61K 9/52, A61K 9/10, A61K 47/00, B01J 13/02, A61K 31/195

(54) **Method and composition for making liposomes**
Verfahren und Zusammensetzung zur Herstellung von Liposomen
Procédé et composition pour la préparation de liposomes

(30) Priority: 07.08.1985 US 763484
(43) Date of publication of application: 25.02.1987
(73) Proprietor: SMITHKLINE BEECHAM CORPORATION, Philadelphia Pennsylvania 19103 (US)
(72) Inventor: Park, John Y., Santa Ana, CA 92705 (US); Thompson, Shurl A., El Toro, CA 92630 (US)
(74) Representative: Hutchins, Michael Richard

(56) References cited:
- EP-A- 0 087 993
- EP-A- 0 092 453
- EP-A- 0 102 324
- EP-A- 0 120 722
- EP-A- 0 130 577
- WO-A-84/02076
- AT-B- 356 278
- CH-A- 498 627
- DE-A- 2 656 333
- DE-A- 2 706 705
- DE-A- 2 856 333
- GB-A- 2 041 871
- GB-A- 2 047 535
- US-A- 3 932 657
- US-A- 3 957 971
- US-A- 4 174 296

## Description

### Background

This invention relates to novel liposome-forming compositions which provide a new method for forming liposomes. More specifically, this invention relates to the use of hydrating agents, compounds with at least two ionizable groups, with liposome-forming materials to make a gel which spontaneously forms liposomes when diluted with an aqueous solution.

### General Description

Numerous processes and methods have been developed for making the different types and sizes of liposomes and for encapsulating active ingredient. Most of these methods have focused on the use of an organic solvent to ensure complete solubilization and uniform mixing of the phospholipids and fatty acids prior to dispersion in an aqueous system. A second development was the use of ultrasonic irradiation to disperse the phospholipid/fatty acid material.

For example Robinson, Trans. Faraday Soc., 56:1260 (1960) and Papahadjopoulos, et al. [Biochim. Biophys. Acta, 135, 639 (1967)] describe formation of phospholipid dispersions from a two-phase ether/water system involving evaporation of the ether by bubbling nitrogen through the mixture. Similarly, chloroform has been used by Chowhan, et al., Biochim. Biophys. Acta, 266:320 (1972) to insure a complete and thorough mixing of the phospholipids prior to dispersion.

Ultrasonic dispersion, first described by D. Papahadjopoulos and N. Miller in Biochim. Biophys. Acta, 135:624 (1967) produces small unilamellar vesicles but the technique is limited because of low encapsulation efficiency.

Batzri and Korn [Biochim. Biophys. Acta, 198:1015 (1973] have used the technique of injecting the lipids in an organic phase (ethanol) into an aqueous solution. Ether was used by Deamer and Bangham in essentially the same technique [Biochim. Biophys. Acta, 443:619 (1976)].

Yet another technique involves a calcium-induced structural change in a lipid vesicle derived from or containing phosphatidylserine but is reported to have a relatively low encapsulation efficiency due to the method of reconstitution of the vesicle. See Papahadjopoulos, et al., Biochim. Biophys. Acta, 394:483 (1975) and H. Hauser, Trends in Pharm., Science 3, 274-77 (1982).

Several other patents set out methods for lipid vesicle formation of interest to this invention. U.S. patent No. 3,804,776 describes a method for producing oil and fat encapsulated amino acids for polypeptides by dispersing dry powders of the material in a molten mixture of the fat or oil and then pouring the mixture into water. The encapsulated material is contained within relatively large droplets of lipid. Such vesicles are not suitable for IV injection and are limited to use only for oral administration.

Entrapment of certain drugs in lipid vesicles by freezing the aqueous phospholipid dispersion of the drug and lipid is described in U.S. patent No. 4,016,100.

Papahadjoplous and Szoka, in U.S. patent No. 4,235,871, disclose a method for making liposomes where the vesicle-forming material is dissolved in an organic solvent and then mixed with an aqueous solution containing the material to be encapsulated. An homogeneous water-in-oil emulsion is formed and the organic solvent is evaporated to give a gel-like mixture. This gel is then suspended in water to form the vesicles.

Another process of interest is disclosed in U.S. patent No. 3,932,657, which teaches the encapsulation of polyaminopolycarboxylic chelating agents, EDTA and TDPA. Yet another U.S. patent, 4,217,344, issued to Vanlerberghe, et al., notes that certain additives can be combined with nonionic lipid compounds so as to modify the permeability and superficial charge of lipid vesicles. Among the several additives mentioned are polypeptides and proteins. Mackaness, et al. describe in U.S. Patent 4,192,859, contrasts media containing liposomes as carriers. The list of contrast agent salts includes arginine salt, cystein salt, glycine salt, glycyl glycine salt, and N-methylglucosamine salts. These materials are characterized as aliphatic and alicyclic amines which can be used to prepare water soluble salts of the various contrast agents which may be employed in X-ray contrast agents.

An article in Science, March, 1984, by Janos Fendler makes reference to a number of synthetic surfactants which may be used in forming vesicles. Fendler references quaternary ammonium and carboxylate, sulfate, sulfonate and phosphate zwitterionic materials which are referenced to the following literature articles: J. H. Fendler, Acc. Chem. Res., 13, 7 (1980): T. Kunitake and S. Shinkai, Adv. Phis. Org. Chem., 17, 435 (1980); T. Kunitake, et. al. J. Am. Chem. Soc., 103, 5401 (1981); J. H. Fuhrhop and J. Matthieu, J. Chem. Soc. Chem. Commun. p. 141 (1983); and W. Talmon, et. al., Science, 221, 1047 (1983).

EP-A-0087993 describes a method of preparing hydrated lipidic lamellar phases by dissolving at least one amphiphilic lipid and a hydrophobic component in an organic solvent such as chloroform or dichloromethane and then atomising the solution to give a finely dividend powder which can thereafter be dispersed in water to give liposomes.

EP-A-0130577 discloses a method of producing liposomes which comprises mixing membrane components such as phospholipids with a water-soluble non-volatile organic solvent and dispersing the mixture in an aqueous medium.

It has now been discovered that liposomes are spontaneously formed when an agueous gel containing a particular liposome-forming material and a hydrating agent as defined in the appended claims is dispersed in an aqueous medium. In accordance with the present invention. therefore, there is provided an aqueous gel composition free of non-physiologically acceptable organic solvents and capable of forming liposomes when dispersed in an aqueous medium which composition comprises:
(a) a liposome-forming material comprising a saturated or unsaturated alkyl C₈-C₂₄ carboxylic acid or amine, and optionally another liposome-forming material:
(b) a physiologically acceptable hydrating agent which is a compound having at least two ionizable groups, one of which is capable of forming an easily dissociative ionic salt which can complex with the ionic functionality of said carboxylic acid or amine, which hydrating agent is inherently incapable of forming liposomes in and of itself, said hydrating agent being present in a molar ratio of between 1:20 and 1:0.05 relative to the liposome-forming material (a):
(c) optionally material to be encapsulated; and
(d) water in an amount up to 300 moles relative to the solids.

Examples of hydrating agents, for the purpose of this invention, are arginine and similar amino acids which have at least one ionizable functionality at both the alpha and omega termini of the molecule. These "hydrating" compounds will have either the same type of ionizable group on the molecule, both cationic, or both anionic, or have ionizable groups of opposite charge. It isn't required that the ionizable groups be on the alpha and omega carbons but such compounds represent the preferred embodiments of this invention.

In practical terms, liposomes formed using this invention are formulated as a "pre-liposome gel" referred to herein as a "gel" wherein the liposome forming material is mixed with an appropriate, concentrated aqueous solution of the hydrating compound. This gel, upon dispersion in an aqueous medium efficiently and spontaneously forms liposomes without solvent evaporation, input of ultrasonic irridation or any of the other means developed to insure proper formation of lipid vesicles, liposomes.

Liposomes made with hydrating agents are more stable than the ones produced by conventional methods, including those formed using organic solvents and ultrasonic energy. Liposome formulations having these hydrating agents suffer none of the solvent removal problems of the current technology nor are the liposomes beset by the non-uniform, destructive forces of ultrasonic irradiation inherent in the older methods.

Additionally, the pre-liposome gel can be dehydrated and stored for a substantial period of time and still be capable of spontaneously forming liposomes upon rehydration.

The pre-liposome gel is extraordinarily stable, stable enough to be autoclaved for sterilization. Furthermore, water-soluble or water-insoluble substances to be encapsulated can be added to the gel and will then be incorporated into the liposomes upon dispersion of the gel. This capability has the effect of greatly enhancing the encapsulation efficiency.

Furthermore, it also has been discovered that the concentration of hydrating agent influences the size of the resulting liposomes in a predictable manner at a given pH. Correspondingly, varying the pH of the dispersing aqueous solution while holding the hydrating agent constant also influences the size of the liposomes produced in a predictable manner.

Thus, the present invention provides an easier, more convenient and predictable means for controlling vesicle size over methods previously available. This method also has no limitations on the concentrations of lipids in the preparation of liposomes.

### SPECIFIC EMBODIMENTS

### Definitions

For the purpose of this invention, a hydrating agent means a compound having at least two ionizable groups, preferably of opposite charge, one of which is capable of forming an easily dissociative ionic salt, which salt can complex with the ionic functionality of the liposome-forming material. The hydrating agent inherently does not form liposomes in and of itself. Such agent will also be physiologically acceptable, i.e., it will not have any untoward or deleterious physiological effect on the host to which it is administered in the context of its use in this invention.

Complexing in this context denotes the formation of dissociative ionic salts where one functionality associates with the ionic functionality of the liposome-forming material and the other functionality has hydrophilic properties which impart water-solubility to the resulting complex.

Hydrated complex means the complex formed between the hydrating agent and the liposome-forming material whereby there is formed a specific semi-crystalline arrangement of molecules. Certain particular, specific spectral data characterize the presence of this complex.

Mixtures of the hydrating agent and liposome-forming materials with certain, discrete amounts of water form a gel-like mass. When in this gel form, the hydrating agent and the liposome-forming material arrange into a "hydrated complex" which is a highly ordered liquid crystal. While the liquid crystal structure varies with pH and amount of hydrating agent, the liquid crystal structure remains. NMR spectroscopy confirms that the crystal structure consists of multilamellar lipid bilayers and hydrophilic layers stacked together in alternating fashion. The ³¹p-NMR spectrum exhibits an anisotropic peak, further confirming the existence of multilamellar bilayers.

The word "liposome" has been proposed and accepted as the term to be used in the scientific literature to describe synthetic, oligolamellar lipid vesicles. Such vesicles are usually comprised of one or more natural or synthetic lipid bilayers surrounding an internal aqueous phase.

Examples of other liposome-forming materials, which can be used in combination with the saturated or unsaturated C₈-C₂₄ carboxylic acid or amine, include saponifiable and non-saponifiable lipids, e.g., the acyl glycerols, the phosphaglycerides, the sphingolipids, the glycolipids, etc., mono-alkyl (C₈∼₂₇) esters of C₄∼C₁₀ dicarboxylic acids (e.g., cholesterol hemi-succinic acid and fatty acid derivatives of amino acids in which any N-acyl carboxylic acids also are included (e.g., N-oleoyl threonine, N-linoleoyl serine), mono- or di-alkyl (C₈∼₂₄) sulfonate esters and mono- or di-alkyl (C₈∼C₂₄) phosphate esters. Furthermore, mono- or di-acyl (C₈∼₂₄) glycerol derivatives of phosphoric acids and mono- or di-acyl (C₈∼C₂₄) glycerol derivatives of sulfuric acids can be used.

Photopolymerizable lipids and/or fatty acids (or amines) (e.g., diacetylenic fatty acids) also can be included, which can provide a sealed liposome with cross-linked membrane bilayers upon photo-initiation of polymerization.

Although the primary components of these liposomes will be fatty acids or fatty amines, there may also be added various other components to modify the liposomes' permeability. There may be added, for example, non-ionic lipid components such as polyoxy alcohol compounds, polyglyceral compounds or esters of polyoles, polyoxyalcolinolated alcohols; the esters of polyoles and synthetic lipolipids, such as cerebrosides. Other materials, such as long chain alcohols and diols, sterols, long chain amines and their quaternary ammonium derivatives; polyoxyethylenated fatty amines, esters of long chain amino alcohols and their salts and quaternary ammonium derivatives; phosphoric esters of fatty alcohols, polypeptides and proteins.

As described hereinabove, the composition of the liposome can be made of more than one component of the various kinds of lipids, in addition to the fatty acids or alkyl amines, and the hydrating agents.

It also has been discovered that the lipid composition may not require the inclusion of the hydrating agents to form the "pre-liposome gel" or liposomes, if the substances (e.g., medicaments, biologically active compounds, cosmetics, etc.) to be encapsulated possess the aforementioned properties. For example, the mixture of DPPC and oleic acid with aqueous epinephrine solution forms the "pre-liposome gel" and liposomes.

For pharmaceutical application as a liposome drug delivery system, however, the composition of phospholipids, oleic acid and arginine or lysine (or glutamic acid and/or aspartic acid) was preferred.

When solids are referred to, the liposome-forming materials, hydrating agents, and material to be encapsulated if any, is what is being referred to.

### Preferred Embodiments

The preferred hydrating agents of this invention are alpha amino acids having an ionizable omega subsitution such as a carboxylate, amino, and guanidino function and those compounds represented by the formula:

X-(CH₂)n-Y I

wherein
X is H₂N-C(NH)-NH-, H₂N-, ZO₃S-, Z₂O₃P-, or ZO₂C- wherein Z is H or an inorganic or organic cation;
Y is -CH(NH₂)-CO₂H, -NH₂, -NH-C(NH)-NH₂, -COOH, CH(NH₂)SO₃Z or ZH(NH₂)PO₃Z₂ wherein Z is defined above; and
n is the integer 1-10; or
a pharmaceutically acceptable salt thereof. Also included in the list of preferred compounds are the N,N'-dialkyl substituted arginine compounds and similar compounds where the alkyl chain length is varied.

More preferred hydrating agents are the omega-substituted, alpha amino acids such as arginine, its N-acyl derivatives, homoarginine, gamma-aminobutyric acid, asparagine, lysine, ornithine, glutamic acid, aspartic acid or a compound represented by the following formulae:

H₂NC(NH)-NH-(CH₂)ₙ-CH(NH₂)COOH II

H₂N-(CH₂)ₙ-CH(NH₂)COOH III

H₂N-(CH₂)ₙ-NH₂ IV

H₂NC(NH)-NH-(CH₂)ₙ-NH-CH(NH)-NH₂ V

HOOC-(CH₂)ₙ-CH(NH₂)COOH VI

HOOC-(CH₂)ₙ-COOH VII

HO₃S-(CH₂)ₙ-CH(NH₂)COOH VIII

H₂O₃S-(CH₂)ₙ-CH(NH₂)COOH IX

HO₃S-(CH₂)ₙ-CH(NH₂)SO₃H X

H₂O₃S-(CH₂)ₙ-CH(NH₂)PO₃H₂ XI

therein n is 2-4.

The most preferred compounds are arginine, homoarginine, gamma-aminobutyric acid, lysine, ornithine, glutamic acid or aspartic acid.

A 1:20 molar ratio of hydrating agent relative to the liposome-forming material will provide the salutory effects of this invention with an upper limit of 1:0.05. The preferred concentration range for the hydrating agent is between a 1:2 to 1:0.5 molar ratio of the hydrating relative to the liposome-forming material.

The hydrating agents of this invention may be used alone or as a mixture. No limitation is implied or intended in the use of mixtures of these hydrating materials.

As a practical matter, thus a matter of preference, if liposomes are prepared with liposome-forming materials having a negative charge, it is preferred to use hydrating agents which contain at least one ionizable nitrogen, such as arginine, homoarginine, lysine, ornithine.

Conversely, if the amphipatic materials used to form the liposomes are nitrogen-based, it is preferred to use a di-acid such as glutamic acid, aspartic acid; any of the alkyl di-acids such as the simple di-acids or those di-acids having two phosphate, or sulfate functionalities; or those di-acids having mixed -COOH/-SO₃H or -COOH/-PO₃H₂ functions.

### Source of hydrating agents

The hydrating agents of this invention are listed in the catalogue of many chemical producers, can be custom manufactured by such producers, or can be made by means known in the art.

Arginine, homoarginine, lysine, glutamic acid, aspartic acid, and other naturally occurring amino acids may be obtained by the hydrolysis of protein and separation of the individual amino acids or from bacterial sources.

The compounds of formula II can be made by the method of Eisele, K. et al, Justusliebigs. Ann. Chem., p 2033 (1975). Further information on several representative examples of these compounds is available through their respective Chemical Abstracts Service numbers as follows: norarginine, CAS # 14191-90-3; arginine, CAS # 74-79-3; and homoarginine, CAS # 151-86-5.

For representative examples of formula III, see for 2,4-diaminobutanoic acid CAS # 305-62-4 and for lysine CAS # 56-87-1.

Methods for making representative compounds of formula IV are available from Chemical Abstracts as follows: ethane diamine, CAS # 305-62-4; propane diamine - 54618-94-9; and 1,4-diaminobutane, CAS # 52165-57-8. See specifically Johnson. T.B., J. Am. Chem. Soc., 38, 1854 (1916).

Of the compounds of formula VI, glutamic acid is well known in the art and is available from many commercial sources. How to make other representative compounds is contained in the literature, for example: 2-aminohexandioic acid - CAS # 62787-49-9 and 2-aminoheptandioic acid - CAS # 32224-51-0.

Glutamic acid, the compound of formula VII where n is 2 is well known in the art and can be made by the method of Maryel and Tuley, Org. Syn, 5, 69 (1925). Other representative compounds in this group can be made according to the art as referenced by the following CAS numbers: hexadioic acid, CAS # 123-04-9 and heptadioic acid, CAS # 111-16-0.

Homocysteic acid is known in the art referenced by CAS # 56892-03-6. The compound 3-sulfovaline is described in the literature referenced by CAS # 23405-34-2.

### Pre-liposome Gel

Mixtures of liposome-forming materials and one or more hydrating agents with up to 300 moles of water relative to the total liposome-forming material gives a gel which focus liposomes directly therefrom upon addition of an aqueous medium. Thin gel is labeled a pre-liposome gel because i.) of its structural characteristics which are essentially those of liposomes and, ii.) the gel's facility for being converted into liposomes upon dilution with an aqueous medium. Aqueous medium in excess of about 300 moles cause the beginning of liposome formation.

The structure of this gel is a highly ordered liquid crystal which forms an optically clear solution. The X, Y, and Z dimensions of the liquid crystal vary with the concentrations of hydrating agent at the constant pH as well as with the pH of the solution. By varying the hydrating agent concentration at constant pH or changing the pH while maintaining percentage of hydrating agent, the size and number of lamellae structures of the lipid bilayers of the subsequent liposome vesicles can be controlled.

The gel structure itself can accommodate up to approximately 300 moles of water per mole of lipid or fatty acid without disturbing the stability of the gel structure. The structure of the gel as determined by proton NMR spectroscopy is comprised of multilamellar lipid bilayers and hydrophilic layers stacked together in an alternating fashion. The ³¹P-NMR spectrum of the same gel exhibits an anisotropic peak further confirming that the gel consists of a multilamellar bilayer.

This gel can be autoclaved, a convenient means of sterilization. Furthermore, the gel shows no discoloration and remains clear at room temperature for at least one year after being autoclaved. The gel can further be sterilized by filtration through an appropriate sterilization filter.

Upon dispersion of the gel into an aqueous medium, liposomes are efficiently and spontaneously produced.

The pre-liposome gel, with or without the material to be encapsulated, also can be dehydrated (lyophilized) and the powder rehydrated to form liposomes spontaneously, even after a long period of storage. This capability makes the invention particularly useful for administering water-sensitive medicaments where long term pre-use storage is needed.

Either water insoluble or water soluble chemicals, drugs, cosmetics, food materials and the like, can be incorporated into liposomes prepared using this material and by this method. Accordingly, the gel may be used as a delivery system for the administration of medicaments via oral, parenteral, topical, intravenous, suppository routes or any other route of drug or chemical delivery.

The use of these liposomes is not limited to human or mammalian use but can be used in any industrial, agricultural, pharmaceutical, cosmetic or chemical application where lipid vesicle encapsulation and administration of compounds or materials is warranted or useful.

The versatility of the present invention is illustrated by the following examples.

### Example #1

### Preparation of Liposomes (Gel Phase)

Dipalmitoylpbosphatidylcholine 3.0 grams, was weighed into a 50 ml beaker. Oleic acid 1.2 grams was added and mixed together to form a uniform paste.

Arginine 0.72 grams in 30 ml of distilled deionized water was added to the dipalmitoylphosphatidylcholine-oleic acid paste and heated to 45°C. With mixing by hand, the mixture formed a clear stable gel. The gel was stored and liposomes later formed by diluting the gel with phosphate buffered saline.

### Example #2

### Preparation of Liposomes

Dipalmitoylphosphatidylcholine, 120 mg, and 24 mg of oleic acid were added together and mixed thoroughly until a white homogeneous paste was observed.

Then 20 mg of arginine was dissolved into 60 ml of phosphate buffered saline (ionic strength = 0.15, pH = 7.4). The arginine-saline solution was added to the paste and heated to 40°C for 1/2 hour, or until a slightly turbid solution was observed.

### Example #3

### Large Scale Gel and Liposome Preparation

i). Gel Manufacture: To 50 grams of egg phosphatide powder type 20 (Ashai ChemicalS) was added 20 grams of oleic acid N.F. Mixing gave a white paste which was cooled to 4°C and ground into a fine powder. This powder was added to an aqueous solution containing 20 grams of arginine and 500 grams of distilled deionized water. The mixture was mixed with a spatula as the solution was heated to about 35°C to help hydrate phospholipids. A homogeneous, slightly yellow gel was formed. This gel can be stored at 4°C or can be frozen and later reconstituted.
ii). Manufacture of Liposomes: The gel prepared in the preceding paragraph was taken from cold storage and returned to room temperature. It was then mixed with 2 liters of phosphate buffered saline, pH 7.4. A white opaque liposome solution was formed.

### Example #4

### Liposome Formation from the Gel

A homogeneous paste of 1.0 gram of dipalmitoylphosphatidylcholine (DPPC) and 400 mg of oleic acid was formed. Then 300 mg of arginine was mixed in 10 ml of phosphate buffered saline, heated to 45°C and added to the DPPC/oleic acid paste to form liposomes.

### Example #5

### Pre-Liposome Gel

One gram of dipalmitoylphosphatidylcholine (DPPL) was mixed with 400 mg of oleic acid to form a homogeneous pasta. 300 mg of arginine was mixed with 2 ml of water at 45°C until dissolved. The arginine solution was mixed with the DPPC/oleic acid paste at about 45°C to give a thick gel. Liposomes formed when this gel was diluted with phosphate buffered saline.

### Example #6

### Cholesterol Containing Liposomes

Cholesterol, 15 mg, was mixed with 100 mg dipalmitoylphosphatidylcholine (DPPC) to form a homogeneous powder. Then 23 mg of oleic acid was added to the powder and thoroughly mixed to form a homogeneous paste. To make liposomes, 30 mg of arginine was added to 10 ml of phosphate buffered saline, heated to 40°C and added to the DPPC/cholesterol/oleic acid paste. The combination was mixed at 40°C to obtain liposomes.

### Example #7

### Palmitic Acid-Containing Liposomes

Dipalmitoylphosphatidylcholine (DPPC) 250 mg was mixed with 25 mg of palmitic acid to form a uniform powder. Then 80 mg of oleic acid was mixed with this powder and heated to 45°C with constant stirring until a uniform paste was formed. Arginine 100 mg was dissolved in 25 ml of distilled deionized water and heated to 45°C. This arginine solution was added to the paste at 45°C and mixed until a uniform homogeneous gel was formed. The gel was diluted ten fold with phosphate buffered saline to form liposomes.

### Example #8

### Isostearic Acid-Containing Liposomes

Dipalmitoylphosphatidylcholine 100 mg was mixed with 50 mg of isostearic acid to form a uniform homogeneous paste. An arginine solution of 50 mg of arginine in 2.0 ml of distilled deionized water was made and added to the isostearic acid paste and heated to 45°C. The mixture was mixed until a clear gel was formed. Liposomes are formed upon dilution with phosphate-buffered saline.

### Example #9

### Oleoyl Threonine Containing Liposomes

Dipalmitoylphosphatidylcholine 125 mg and 75 mg of oleoyl threonine were added together and heated to 40° C to form a paste. Then 2 ml of distilled deionized water was added with constant mixing at 40°C. A clear gel was formed which can be diluted with phosphate buffer saline at pH 5 to form liposomes.

### Example #10

### Myristyl Amine Containing Liposomes

Dipalmitoylphosphatidylcholine 192 mg was added to 72 mg of myristyl amine and heated with constant mixing until a uniform paste was formed. Glutamic acid 65 mg in 5 ml of distilled deionized water was added to the paste and heated until a gel was formed. Phosphate buffered saline was added to the gel to form liposomes.

### Example #11

### DLPC Containing Liposomes

Dilaurylphosphatidylcholine (DLPC) 50 mg was mixed with 20 mg oleic acid to form a homogeneous paste. Arginine 20 mg was added to 10 ml of phosphate buffered saline, added to the paste and hand mixed until a turbid liposome solution formed.

### Example # 12

### Phosphatidylethanolamine-Glutamic Acid Liposomes

L-glutamic acid 32 mg was dissolved in 2.0 ml of distilled deionized water and the pH adjusted to 5.2 with 1.0 N sodium hydroxide. This solution was heated to 60°C, and 100 mg of phosphatidylethanolamine added. The solution was kept at 60°C with constant mixing until a uniform viscous gel was observed.

The phosphatidylethanolamine-glutamic acid gel was diluted 1/10 by phosphate buffered saline. Vesicular like structures are observed under phase contrast light microscopy.

### Example #13

### Dipivalylepinephrine Liposomes

One gram of dipalmitoylphosphatidylcholine was mixed with 396 mg of oleic acid, until a homogeneous paste formed. Then 400 mg of arginine in 20 ml of distilled deionized water was added to form a pre-vesicle clear gel.

To make the liposomes, 242 mg of dipivalylepinephrine was dissolved in 10 ml of distilled deionized water. Then 5 grams of the pre-vesicle gel was mixed with 5 grams of the dipivalylepinephrine solution after which 50 ml of phosphate buffered saline was added forming a liposome solution.

### Example #14

### Flurbiprofen Liposomes

To make these liposomes, 980 mg of dipalmitoylphosphatidylcholine, 370 mg of oleic acid, and 320 mg of flurbiprofen (free acid) were mixed together until a homogeneous paste was observed. Then 510 mg of arginine in 10 ml of purified water was added to the paste and heated to 41°C with constant mixing for 30 minutes. A clear pre-vesicle gel formed of which 5 grams was introduced into 50 ml of phosphate buffered saline and mixed with a stir bar until a bluish translucent solution was observed.

### Example #15

### Levobunolol Liposomes

Thirty mg of dipalmitoylphosphatidylcholine and 15 mg of cholesterol were weighed into a 4 ml vial. Ten mg of linoleic acid was added and mixed together to form a uniform paste. Two ml of a 1% aqueous levobunolol solution containing 10 mg of arginine was added to the paste and mixed together. Then 10 ml of phosphate buffer solution was added and heated to 45°C to form liposomes.

### Example #16

### Pilocarpine Liposomes

To 120 mg of dipalmitoylphosphatidylcholine was added 40 mg of oleic acid to form a homogeneous paste. Forty mg of pilocarpine free base was added to 10 ml of distilled deionized water. This solution was added to the paste and heated to 45°C to form a pre-liposome gel. The resulting gel was diluted with 20 ml of phosphate buffered saline to form liposomes.

### Example #17

### Epinephrine Liposomes

Dipalmitoylphosphatidylcholine 250 mg was mixed with 100 mg of oleic acid to form a homogeneous paste. 50 mg of epinephrine, free base, was dissolved in 5.0 ml of distilled deionized water, heated to 40°C and added to the dipalmitoylphosphatidylcholine/oleic acid paste. This solution was mixed until a homogeneous viscous creamy gel was observed. This gel was diluted 1/5 with phosphate buffered saline (pH 7.22) to form liposomes.

### EXAMPLE #18

### Effect of Arginine Concentration on Liposome Size

To 502 mg of dipalmitoylphosphatidylcholine (DPPC) was added 10 microliters of (2-palmitoyl-1-C¹⁴) (0.1 mCi/ml) dipalmitoylphosphatidylcholine. Chloroform was added to effect complete mixing of the radioactivity and then evaporated. Oleic acid (OA), 195 mg, was then mixed into the lipid to form a paste. Five ml of distilled water containing 119 mg of arginine was added and mixed at 45°C to form a clear gel.

One gram of the gel was weighed into four different vials and arginine was added as follows:

| Sample ID | Sample Composition DPPC:OA:Arg |
|---|---|
| Vial 1 + 1 ml water | (1:1:1) |
| Vial 2 + 1 ml of 50 mg/ml Arg in H₂O | (1:1:3) |
| Vial 3 + 1 ml of 84 mg/ml Arg in H₂O | (1:1:5) |
| Vial 4 + 1 ml of 192 mg/ml Arg in H₂O | (1:1:10) |

One-half gram of each solution was diluted in 50 ml of phosphate buffered saline of pH 7.8.

The estimated weight diameter was obtained from a Sephracyl S-1000 column chromatographic analysis employing ¹⁴C-isotope labelled DPPC. The effects are given in the following Table.

**Table II**

| Effects of Arginine Concentration on Vesicle Size | | |
|---|---|---|
| System | pH | Estimated Weight Diameter (nm) |
| DPPC:OA:Arg (1 :1: 1) | 7.8 | ∼220 |
| DPPC:OA:Arg (1 :1: 3) | 7.8 | ∼140 |
| DPPC:OA:Arg (1 :1: 5) | 7.8 | ∼90 |
| DPPC:OA:Arg (1 :1: 10) | 7.8 | ∼20 |

### EXAMPLE #19

### PH Effect on Vesicle Size

Additionally, the vesicle size can be varied by varying the pH of the aqueous buffer solution.

To 100 mg of diplamitoylphosphatidylcholine (DPPC) was added 25 microliters of (2-palmitoyl-1-C¹⁴)(0.1 mCi/ml) dipalmitoylphosphatidylcholine. Chloroform was added to effect complete mixing of the radioactivity and then evaporated. Oleic acid (OA), 40.1 mg, was then mixed into the lipid to form a paste. One ml of a solution containing 24 mg/ml arginine in water was added to the lipid mixture and mixed at 45°C to form a clear gel.

Two 100 mg aliquots of this gel were diluted in 10 ml of phosphate buffer at pH 9.0 and 7.4 respectively.

Again, the estimated weight diameter (A) was obtained from the Sephracyl S-1000 column chromatographic analysis employing ¹⁴C-isotope labelled dipalmitoylphosphotidylcholine. Results are given in the following Table.

**Table III**

| pH Effects on Vesicle Size | | |
|---|---|---|
| System | Estimated Weight | |
| | pH | Diameter (nm) |
| DPPC:OA:Arg (1 :1: 1) | 7.4 | ∼>300 |
| DPPC:OA:Arg (1 :1: 1) | 7.8 | ∼220 |
| DPPC:OA:Arg (1: 1 :1) | 9.0 | ∼25.4 |

Thus, a desired size of the liposomal vesicles can be prepared by varying the arginine concentration or the pH of the aqueous buffer solution.

### EXAMPLE #20

### Liposome Stability

Sterile liposomes may be prepared from the heat sterilized pre-liposome gel. Alternatively, the liposome gel or the liposomes may be sterile filtered through an appropriate sterilizing filter.

Liposomes prepared from DPPC:OA:Arg (1:1:2) at pH 8.0 were heat sterilized and stored at room temperature for approximately one year without adding antimicrobial agents and anti-oxidants. No bacterial growth, discoloration and precipitation were observed. Negative stain electron microscopic examination of the one year old liposomes revealed that the liposomal vesicles are stable.

### EXAMPLE #21

Encapsulated sucrose latency was measured using C¹⁴-sucrose encapsulated with the DPPC:OA:Arg (1:1:1) liposome system in aqueous phosphate buffer solution at pH 7.8. The result was presented in Table IV.

**Table IV**

| % Sucrose Latency | |
|---|---|
| Days | % Latency |
| 0 | 100 |
| 1 | 97.4 |
| 3 | 93.4 |
| 7 | 91.4 |

Thus, the present liposome system has an excellent latency for drug delivery.

### EXAMPLE #22

### Efficiency of Encapsulation

A number of drugs were encapsulated with 10 mg/ml DPPC:oleic acid:Arg (1:1:1) liposomes to illustrate medicament encapsulation for use as a drug delivery system. The results are presented in Table V.

**Table V**

| Entrapment of Drugs | | |
|---|---|---|
| Drugs | pH | % Entrapment |
| Flurbiprofen | 7.8 PBS | 90% |
| Dipivalyl Epinephrine | 7.1 PBS | 80% |

### Example #23

### Lyophilized Liposomes

Oleic acid, 30.0 gm, and 7.5 gm of cholesterol U.S.P. were confected. Then 75.0 gm of phosphatide type 20 powder (Asahi Chemical Co.) was mixed with the oleic acid/cholesterol mixture until an homogeneous paste was formed.

Then 15.0 gm of arginine (free base) was dissolved in 183 gm of distilled, deionized water. This arginine solution was mixed slowly with the lipid paste to form a homogeneous gel. The gel pH was adjusted to 7.4 using 5.0 N HCl.

A 10.0 gm aliquot of this pre-liposome gel was transferred to a 10 ml vial and lyophilized. The resulting powder formed liposomes when diluted with 5 ml of phosphate buffered saline.

### EXAMPLE #24

### Acid Stable Liposome Preparations

An example of acid stable liposomes employing this invention is illustrated by liposomes prepared with the following materials: distearoylphosphatidylcholine dipalmitoyl phosphatidylcholine: oleic acid, arginine, and cholesterol. These materials were combined in a molar ratio of 1:2:2:2:0.2 as follows: Cholesterol, 20 mg., was mixed with 144 mg. of oleic acid and treated to 40°C. DSPC, 200 mg., and 400 mg. of DPPC was added and mixed at 40°C. The mixture was stirred until a uniform homogeneous paste was formed.

Arginine, 88 mg., was dissolved in 1.15 g. of deionized distilled water. This arginine solution was added to the lipid paste and mixed at about 45°C until a homogeneous pre-liposome gel formed. The pH of the gel was adjusted to various pH levels with 0.1N HCl. The gel was diluted 10-fold with 0.9% NaCl, forming vesicles.

| Liposome Stability at pH 4.4 | |
|---|---|
| Time/Days | Size/(NMx10³) |
| 0 | 1.024 |
| 3 | 1.136 |
| 7 | 1.127 |

## Claims (Claims for the following Contracting State(s): BE, CH, DE, FR, GB, IT, LI, LU, NL, SE)

1. An aqueous gel composition free of non-physiologically acceptable organic solvents and capable of forming liposomes when dispersed in an aqueous medium, which composition comprises:
(a) a liposome-forming material comprising a saturated or unsaturated alkyl C₈-C₂₄ carboxylic acid or amine, and optionally another liposome-forming material;
(b) a physiologically acceptable hydrating agent which is a compound having at least two ionizable groups, one of which is capable of forming an easily dissociative ionic salt which can complex with the ionic functionality of said carboxylic acid or amine, which hydrating agent is inherently incapable of forming liposomes in and of itself, said hydrating agent being present in a molar ratio of between 1:20 and 1:0.05 relative to the liposome-forming material (a);
(c) optionally material to be encapsulated; and
(d) water in an amount up to 300 moles relative to the solids.

2. The composition of claim 1 wherein the hydrating agent is an alpha amino acid having an omega substitution which is a carboxylate, amino, or guanidino function or a pharmaceutically acceptable salt thereof, or a compound of the formula:
X-(CH₂)ₙ-Y I
wherein
X is H₂N-C(NH)-NH-, H₂N-, ZO₃S-, Z₂O₃P-, or ZO₂C- wherein Z is H or an inorganic or organic cation;
Y is -CH(NH₂)-CO₂H, -NH₂, -NH-C(NH)-NH₂, -COOH, CH(NH₂)SO₃Z or ZH(NH₂)PO₃Z₂ wherein Z is defined above; and
n is the integer 1-10; or
a pharmaceutically acceptable salt thereof.

3. The composition of claim 2 wherein the hydrating agent is present in an amount between 1:2 to 1:0.5 molar ratio relative to the liposome-forming material (a).

4. The product of claim 3 wherein said hydrating agent is arginine, homoarginine, or their N-acyl derivatives, gamma-aminobutyric acid, asparagine, lysine, ornithine, glutamic acid, aspartic acid or a compound of the formulae:
H₂NC(NH)-NH-(CH₂)ₙ-CH(NH₂)COOH II,
H₂N-(CH₂)ₙ-CH(NH₂)COOH III,
H₂N-(CH₂)ₙ-NH₂ IV,
H₂NC(NH)-NH-(CH₂)ₙ-NH-CH(NH)-NH₂ V,
HOOC-(CH₂)ₙ-CH(NH₂)COOH VI,
HOOC-(CH₂)ₙ-COOH VII,
HO₃S-(CH₂)ₙ-CH(NH₂)COOH VIII,
H₂O₃S-(CH₂)ₙ-CH(NH₂)COOH IX,
HO₃S-(CH₂)ₙ-CH(NH₂)SO₃H X, or
H₂O₃S-(CH₂)ₙ-CH(NH₂)PO₃H₂ XI
wherein n is 2-4, or a pharmaceutically acceptable salt thereof.

5. The composition of claim 4 wherein said hydrating agent is arginine, homoarginine, gamma-aminobutyric acid, lysine, or ornithine or a pharmaceutically acceptable salt thereof.

6. The composition of claim 4 wherein said hydrating agent is glutamic acid or aspartic acid or a pharmaceutically acceptable salt thereof.

7. A process for forming stable liposomes, which comprises dispersing in an aqueous medium in a manner adequate to form liposomes an aqueous gel composition as defined in any one of the preceding Claims.

## Claims (Claims for the following Contracting State(s): AT)

1. A method for making an aqueous gel composition which can be dispersed in an aqueous medium to form liposomes, the composition being free of non-physiologically acceptable solvents, and comprising:
(a) a liposome-forming material comprising a saturated or unsaturated alkyl C₈-C₂₄ carboxylic acid or amine, and optionally another liposome-forming material;
(b) a physiologically acceptable hydrating agent which is a compound having at least two ionizable groups, one of which is capable of forming an easily dissociative ionic salt which can complex with the ionic functionality of said carboxylic acid or amine, which hydrating agent is inherently incapable of forming liposomes in and of itself, said hydrating agent being present in a molar ratio of between 1:20 and 1:0.05 relative to the liposome-forming material (a);
(c) optionally material to be encapsulated; and
(d) water in an amount up to 300moles relative to the solids, wherein the process comprises mixing the liposome-forming material and hydrating agent with the water to form the gel.

2. The method of claim 1 wherein the hydrating agent is an alpha amino acid having an omega substitution which is a carboxylate, amino, guanidino function or a pharmaceutically acceptable salt thereof or a compound of the formula
X-(CH₂)ₙ-Y I
wherein
X is H₂N-C(NH)-NH-, H₂N-, ZO₃S-, Z₂O₃P-, or ZO₂C- wherein Z is H or an inorganic or organic cation;
Y is -CH(NH₂)-CO₂H, -NH₂, -NH-C(NH)-NH₂, -COOH, CH(NH₂)SO₃Z or ZH(NH₂)PO₃Z₂ wherein Z is defined above; and
n is the integer 1-10; or
a pharmaceutically acceptable salt thereof.

3. The method of claim 2 wherein the hydrating agent is present in an amount between 1:2 to 1:0.5 molar ratio relative to the liposome-forming material (a).

4. The method of claim 3 wherein said hydrating agent is arginine, homoarginine, or their N-acyl derivatives, gamma-aminobutyric acid, asparagine, lysine, ornithine, glutamic acid, aspartic acid or a compound of the formulae:
H₂NC(NH)-NH-(CH₂)ₙ-CH(NH₂)COOH II,
H₂N-(CH₂)ₙ-CH(NH₂)COOH III,
H₂N-(CH₂)ₙ-NH₂ IV,
H₂NC(NH)-NH-(CH₂)ₙ-NH-CH(NH)-NH₂ V,
HOOC-(CH₂)ₙ-CH(NH₂)COOH VI,
HOOC-(CH₂)ₙ-COOH VII,
HO₃S-(CH₂)ₙ-CH(NH₂)COOH VIII,
H₂O₃S-(CH₂)ₙ-CH(NH₂)COOH IX,
HO₃S-(CH₂)ₙ-CH(NH₂)SO₃H X, or
H₂O₃S-(CH₂)ₙ-CH(NH₂)PO₃H₂ XI
wherein n is 2-4, or a pharmaceutically acceptable salt thereof.

5. The method of Claim 4 wherein said hydrating agent is arginine, homoarginine, gamma-aminobutyric acid, lysine, or ornithine or a pharmaceutically acceptable salt thereof.

6. The method of Claim 4 wherein said hydrating agent is glutamic acid or aspartic acid or a pharmaceutically acceptable salt thereof.

7. A method for making a liposome composition which method comprises dispersing in an aqueous medium in a manner adequate to form liposomes, an aqueous gel composition as defined in any one of the preceding Claims.

## Patentansprüche (Patentansprüche für folgende(n) Vertragsstaat(en): BE, CH, DE, FR, GB, IT, LI, LU, NL, SE)

1. Wäßrige Gelzusammensetzung, die frei von physiologisch nicht verträglichen organischen Lösungsmitteln ist und bei Dispersion in einem wäßrigen Medium Liposomen zu bilden vermag, die genannte Zusammensetzung umfassend:
(a) ein liposombildendes Material, welches eine gesättigte oder ungesättigte C₈-C₂₄ Carboxylsäure
oder Amin enthält, und ggf. ein anderes liposombildendes Material;
(b) ein physiologisch verträgliches Hydratationsmittel, welches eine Verbindung mit wenigstens zwei ionisierbaren Gruppen ist, von welchen eine ein leicht dissoziatives ionisches Salz zu bilden vermag, das mit der ionischen Funktion der genannten Carboxylsäure oder des Amins einen Komplex bilden kann, wobei das Hydratationsmittel von Haus aus an und für sich keine Liposomen zu bilden vermag und wobei das Hydratationsmittel in einem Molarverhältnis zwischen 1:20 und 1:0,05 relativ bezogen auf das liposombildende Material (a) vorliegt;
(c) ggf. ein zu kapselndes Material; sowie
(d) Wasser in einer Menge von bis zu 300 Mol relativ bezüglich den Feststoffen.

2. Zusammensetzung nach Anspruch 1, bei welcher das Hydratationsmittel eine Alphaaminosäure mit einer Omegasubstitution, welche eine Carboxylat-, Amino- oder Guanidinofunktion ist, oder ein pharmazeutisch verträgliches Salz hiervon ist, oder eine Verbindung der Formel
X-(CH₂)ₙ-Y I
worin
X H₂N-C(NH)-NH-, H₂N-, ZO₃S-,
Z₂O₃P-, oder ZO₂C- ist, worin Z H
oder ein anorganisches oder organisches Kation ist;
Y -CH(MH₂)-CO₂H, -NH₂,
-NH-C(NH)-NH₂, -COOH, CH(NH₂)SO₃Z
oder ZH(NH₂)PO₃Z₂ ist, worin
Z die oben definierte Bedeutung hat; und
n eine ganze Zahl von 1-10 ist; oder
ein pharmazeutisch verträgliches Salz hiervon ist.

3. Zusammensetzung nach Anspruch 2, bei welcher das Hydratationsmittel in einer Menge entsprechend einem Molarverhältnis zwischen 1:2 bis 1:0,5 relativ bezüglich dem liposombildenden Material (a) vorliegt.

4. Das Erzeugnis nach Anspruch 3, bei welchem das genannte Hydratationsmittel Arginin, Homoarginin, oder ihre N-Acyl Derivate, Aminobuttersäure, Asparagin, Lysin, Ornithin, Glutaminsäure, Asparaginsäure oder eine Verbindung gemäß den Formeln
H₂NC(NH)-NH-(CH₂)ₙ-CH(NH₂) COOH II
H₂N-(CH₂)ₙ-CH(NH₂) COOH III
H₂N-(CH₂)ₙ-NH₂ COOH IV
H₂NC(NH)-NH-(CH₂)ₙ-NH-CH(NH)-NH₂ V
HOOC-(CH₂)ₙ-CH(NH₂) VI
HOOC-(CH₂)ₙ- COOH VII
HO₃S-(CH₂)ₙ-CH(NH₂)COOH VIII
H₂O₃S-(CH₂)ₙ-CH(NH₂)COOH IX
HO₃S-(CH₂)ₙ-CH(NH₂)SO₃H X
oder
H₂O₃S-(CH₂)ₙ-CH(NH₂) PO₃H₂ XI
wobei n einen Wert von 2-4 besitzt, oder
ein pharmazeutisch verträgliches Salz hiervon ist.

5. Zusammensetzung nach Anspruch 4, bei welcher das genannte Hydratationsmittel Arginin, Homoarginin, Gammaaminobuttersäure, Lysin oder Ornithin oder ein pharmazeutisch verträgliches Salz hiervon ist.

6. Zusammensetzung nach Anspruch 4, wobei das genannte Hydratationsmittel Glutaminsäure oder Asparaginsäure oder ein pharmazeutisch verträgliches Salz hiervon ist.

7. Verfahren zur Bildung stabiler Lyposome, umfassend das Dispergieren einer wäßrigen Gelzusammensetzung gemäß einem der vorhergehenden Ansprüche in einem wäßrigen Medium in einer zur Bildung von Liposomen geeigneten Weise.

## Patentansprüche (Patentansprüche für folgende(n) Vertragsstaat(en): AT)

1. Verfahren zur Herstellung einer wäßrigen Gel-Zusammensetzung, die in einem wäßrigen Medium zur Bildung von Liposomen dispergiert werden kann, wobei die Zusammensetzung frei von physiologisch nicht verträglichen Lösungsmitteln ist und umfaßt:
(a) ein liposombildendes Material, welches eine gesättigte oder ungesättigte C₈-C₂₄ Carboxylsäure
oder Amin enthält, und ggf. ein anderes liposombildendes Material;
(b) ein physiologisch verträgliches Hydratationsmittel, welches eine Verbindung mit wenigstens zwei ionisierbaren Gruppen ist, von welchen eine ein leicht dissoziatives ionisches Salz zu bilden vermag, das mit der ionischen Funktion der genannten Carboxylsäure oder des Amins einen Komplex bilden kann, wobei das Hydratationsmittel von Haus aus an und für sich keine Liposomen zu bilden vermag und wobei das Hydratationsmittel in einem Molarverhältnis zwischen 1:20 und 1:0,05 relativ bezogen auf das liposombildende Material (a) vorliegt;
(c) ggf. ein zu kapselndes Material; sowie
(d) Wasser in einer Menge von bis zu 300 Mol relativ bezüglich den Feststoffen,
wobei das Verfahren das Mischen des liposomenbildenden Materials und des Hydratationsmittels mit dem Wasser zur Bildung des Gels umfaßt.

2. Verfahren nach Anspruch 1, bei welchem das Hydratationsmittel eine Alpha-Aminosäure mit einer Omegasubstitution, welche eine Carboxylat-, Amino- oder Guanidinofunktion ist, oder ein pharmazeutisch verträgliches Salz hiervon ist, oder eine Verbindung der Formel
X-(CH₂)ₙ-Y I
worin
X H₂N-C(NH)-NH-, H₂N-, ZO₃S-,
Z₂O₃P-, oder ZO₂C- ist, worin Z H
oder ein anorganisches oder organisches Kation ist;
Y -CH(NH₂)-CO₂H, -NH₂,
-NH-C(NH)-NH₂, -COOH, CH(NH₂)SO₃Z
oder ZH(NH₂)PO₃Z₂ ist, worin
Z die oben definierte Bedeutung hat; und
n eine ganze Zahl von 1-10 ist; oder
ein pharmazeutisch verträgliches Salz hiervon ist.

3. Verfahren nach Anspruch 2, bei welchem das Hydratationsmittel in einer Menge entsprechend einem Molarverhältnis zwischen 1:2 bis 1:0,5 relativ bezüglich dem liposombildenden Material (a) vorliegt.

4. Das Verfahren nach Anspruch 3, bei welchem das genannte Hydratationsmittel Arginin, Homoarginin, oder ihre N-Acyl Derivate, Aminobuttersäure, Asparagin, Lysin, Ornithin, Glutaminsäure, Asparaginsäure oder eine Verbindung gemäß den Formeln
H₂NC(NH)-NH-(CH₂)ₙ-CH(NH₂) COOH II
H₂N-(CH₂)ₙ-CH(NH₂) COOH III
H₂N-(CH₂)ₙ-NH₂ COOH IV
H₂NC(NH)-NH-(CH₂)ₙ-NH-CH(NH)-NH₂ V
HOOC-(CH₂)ₙ-CH(NH₂) VI
HOOC-(CH₂)ₙ- COOH VII
HO₃S-(CH₂)ₙ-CH(NH₂)COOH VIII
H₂O₃S-(CH₂)ₙ-CH(NH₂)COOH IX
HO₃S-(CH₂)ₙ-CH(NH₂)SO₃H X
oder
H₂O₃S-(CH₂)ₙ-CH(NH₂) PO₃H₂ XI
wobei n einen Wert von 2-4 besitzt, oder
ein pharmazeutisch verträgliches Salz hiervon ist.

5. Verfahren nach Anspruch 4, bei welchem das genannte Hydratationsmittel Arginin, Homoarginin, Gammaaminobuttersäure, Lysin oder Ornithin oder ein pharmazeutisch verträgliches Salz hiervon ist.

6. Verfahren nach Anspruch 4, wobei das genannte Hydratatationsmittel Glutaminsäure oder Asparaginsäure oder ein pharmazeutisch verträgliches Salz hiervon ist.

7. Verfahren zur Herstellung einer Liposomen-Zusammensetzung, wobei das Verfahren das Dispergieren einer wäßrigen Gelzusammensetzung gemäß einem der vorhergehenden Ansprüche in einem wäßrigen Medium in einer zur Bildung von Liposomen geeigneten Weise umfaßt.

## Revendications (Revendications pour l'(les) Etat(s) contractant(s) suivant(s): BE, CH, DE, FR, GB, IT, LI, LU, NL, SE)

1. Composition de gel aqueux, exempte de solvants organiques non acceptables physiologiquement et capable de former des liposomes lorsqu'elle est mise en dispersion dans un milieu aqueux, laquelle composition comprend:
a) une substance formant des liposomes, comprenant un acide alkylcarboxylique en C₈-C₂₄ saturé ou insaturé ou une amine et éventuellement une autre substance formant des liposomes;
b) un agent d'hydratatation acceptable physiologiquement, qui est un composé comportant au moins deux groupements ionisables dont l'un est capable de former par dissociation un sel ionique qui peut se complexer avec la fonctionnalité ionique dudit acide carboxylique ou de ladite amine, lequel agent d'hydratation est par inhérence incapable de former des liposomes en et par lui-même, ledit agent d'hydratation étant présent dans un rapport molaire compris entre 1:20 et 1:0,5 par rapport à la substance (a) formant des liposomes;
c) éventuellement la substance à encapsuler; et
d) de l'eau dans une proportion pouvant atteindre 300 moles par rapport aux matières solide.

2. Composition selon la revendication 1, dans laquelle l'agent d'hydratation est un α-amino-acide présentant une substitution oméga qui est une fonction carboxylate, amino ou guanidino, ou un sel pharmaceutiquement acceptable de celui-ci, ou un composé de formule
X-(CH₂)ₙ-Y (I)
dans laquelle
X est mis pour H₂N-C-(NH)-NH-, H₂N-, ZO₃S-, Z₂O₃P- ou ZO₂C-, où Z est un atome d'hydrogène ou un cation inorganique ou organique;
Y est mis pour -CH(NH₂)-CO₂H, -NH₂, -NH-C(NH)-NH₂, -COOH, -CH(NH₂)SO₃Z ou -ZH(NH₂)PO₃Z₂, où Z est tel que defini ci-dessus; et
n est un nombre entier de 1 à 10,
ou un sel pharmaceutiquement acceptable de ce composé.

3. Composition selon la revendication 2, dans laquelle l'agent d'hydratation est présent dans un rapport molaire compris entre 1:2 et 1:0,5 par rapport à la substance (a) formant des liposomes.

4. Produit selon la revendication 3, dans lequel ledit agent d'hydratation est l'arginine, l'homoarginine ou leurs dérivés N-acylés, l'acide γ-aminobutyrique, l'asparagine, la lysine, l'ornithine, l'acide glutamique, l'acide aspartique ou un composé de formule
H₂NC(NH)-NH-(CH₂)ₙ-CH(NH₂)COOH II
H₂N-(CH₂)ₙ-CH(NH₂)COOH III
H₂N-(CH₂)ₙ-NH₂ IV
H₂NC(NH)-NH-(CH₂)ₙ-NH-CH(NH)-NH₂ V
HOOC-(CH₂)ₙ-CH(NH₂)COOH VI
HOOC-(CH₂)ₙ-COOH VII
HO₃S-(CH₂)ₙ-CH(NH₂)COOH VIII
H₂O₃S-(CH₂)ₙ-CH(NH₂)COOH IX
HO₃S-(CH₂)ₙ-CH(NH₂)SO₃H X ou
H₂O₃S-(CH₂)ₙ-CH(NH₂)PO₃H₂ XI
dans laquelle n est un nombre de 2 à 4, ou un sel pharmaceutiquement acceptable de ceux-ci.

5. Composition selon la revendication 4, dans laquelle ledit agent d'hydratation est l'arginine, l'homoarginine, l'acide γ-aminobutyrique, la lysine, l'ornithine ou un sel pharmaceutiquement acceptable de ceux-ci.

6. Composition selon la revendication 4, dans laquelle ledit agent d'hydratation est l'acide glutamique, l'acide aspartique ou un sel pharmaceutiquement acceptable de ceux-ci.

7. Procédé pour la formation de liposomes stables, comprenant l'opération consistant à disperser dans un milieu aqueux, de manière adéquate pour former des liposomes, une composition de gel aqueux selon l'une quelconque des revendications 1 à 6.

## Revendications (Revendications pour l'(les) Etat(s) contractant(s) suivant(s): AT)

1. Procédé de preparation d'une composition de gel aqueux qui peut être dispersée dans un milieu aqueux pour former des liposomes, la composition étant exempte de solvants organiques non acceptables physiologiquement et comprenant:
a) une substance formant des liposomes, comprenant un acide alkylcarboxylique en C₈-C₂₄ saturé ou insaturé ou une amine et éventuellement une autre substance formant des liposomes;
b) un agent d'hydratatation acceptable physiologiquement, qui est un composé comportant au moins deux groupements ionisables dont l'un est capable de former par dissociation un sel ionique qui peut se complexer avec la fonctionnalité ionique dudit acide carboxylique ou de ladite amine, lequel agent d'hydratation est par inhérence incapable de former des liposomes en et par lui-même, ledit agent d'hydratation étant présent dans un rapport molaire compris entre 1:20 et 1:0,5 par rapport à la substance (a) formant des liposomes;
c) éventuellement la substance à encapsuler; et
d) de l'eau dans une proportion pouvant atteindre 300 moles par rapport aux matières solide
ledit procédé comprenant l'opération consistant à mélanger la substance formant des liposomes et l'agent d'hydratation avec l'eau pour former le gel

2. Procédé selon la revendication 1, dans lequel l'agent d'hydratation est un α-amino-acide présentant une substitution oméga qui est une fonction carboxylate, amino ou guanidino, ou un sel pharmaceutiquement acceptable de celui-ci, ou un composé de formule
X-(CH₂)ₙ-Y (I)
dans laquelle
X est mis pour H₂N-C-(NH)-NH-, H₂N-, ZO₃S-, Z₂O₃P- ou ZO₂C-, où Z est un atome d'hydrogène ou un cation inorganique ou organique;
Y est mis pour -CH(NH₂)-CO₂H, -NH₂-, -NH-C(NH)-NH₂, -COOH, -CH(NH₂)SO₃Z ou -ZH(NH₂)PO₃Z₂, où Z est tel que défini ci-dessus; et
n est un nombre entier de 1 à 10,
ou un sel pharmaceutiquement acceptable de ce composé.

3. Procédé selon la revendication 2, dans lequel l'agent d'hydratation est présent dans un rapport molaire compris entre 1:2 et 1:0,5 par rapport à la substance (a) formant des liposomes.

4. Procédé selon la revendication 3, dans lequel ledit agent d'hydratation est l'arginine, l'homoarginine ou leurs dérivés N-acylés, l'acide γ-aminobutyrique, l'asparagine, la lysine, l'ornithine, l'acide glutamique, l'acide aspartique ou un composé de formule
H₂NC(NH)-NH-(CH₂)ₙ-CH(NH₂)COOH II
H₂N-(CH₂)ₙ-CH(NH₂)COOH III
H₂N-(CH₂)ₙ-NH₂ (IV)
H₂NC(NH)-NH-(CH₂)ₙ-NH-CH(NH)-NH₂ V
HOOC-(CH₂)ₙ-CH(NH₂)COOH VI
HOOC-(CH₂)ₙ-COOH VII
HO₃S-(CH₂)ₙ-CH(NH₂)COOH VIII
H₂O₃S-(CH₂)ₙ-CH(NH₂)COOH IX
HO₃S-(CH₂)ₙ-CH(NH₂)SO₃H X ou
H₂O₃S-(CH₂)ₙ-CH(NH₂)PO₃H₂ XI
dans laquelle n est un nombre de 2 à 4, ou un sel pharmaceutiquement acceptable de ceux-ci.

5. Procédé selon la revendication 4, dans lequel ledit agent d'hydratation est l'arginine, l'homoarginine, l'acide γ-aminobutyrique, la lysine, l'ornithine ou un sel pharmaceutiquement acceptable de ceux-ci.

6. Procédé selon la revendication 4, dans lequel ledit agent d'hydratation est l'acide glutamique, l'acide aspartique ou un sel pharmaceutique-ment acceptable de ceux-ci.

7. Procédé de préparation d'une composition de liposomes, comprenant l'opération consistant à disperser dans un milieu aqueux, de manière adéquate pour former des liposomes, une composition de gel aqueux selon l'une quelconque des revendications 1 à 6.
